(11) Publication number: **0 181 117 B1**

(12) **EUROPEAN PATENT SPECIFICATION**

(45) Date of publication of patent specification: **19.11.92**

(51) Int. Cl.[5]: **A61K 39/295**, A61K 39/235, A61K 39/29, A61K 39/12, //C12N15/86

(21) Application number: **85307634.7**

(22) Date of filing: **23.10.85**

(54) **Oral vaccines.**

(30) Priority: **01.11.84 US 667233**
**04.10.85 US 782638**

(43) Date of publication of application:
**14.05.86 Bulletin 86/20**

(45) Publication of the grant of the patent:
**19.11.92 Bulletin 92/47**

(84) Designated Contracting States:
**AT BE CH DE FR IT LI LU NL SE**

(56) References cited:
**EP-A- 0 062 574**
**EP-A- 0 185 573**
**WO-A-83/02393**

**NATURE, vol. 302, no. 5908, April 1983, pages 490-495, MacMillan Journals Ltd., Chesham, Bucks, GB; G.L. SMITH et al.: "Infectious vaccinia virus recombinants that express hepatitis B virus surface antigen"**

(73) Proprietor: **AMERICAN HOME PRODUCTS CORPORATION**
**685, Third Avenue**
**New York, New York 10017(US)**

(72) Inventor: **Davis, Alan Robert**
**619 Mallard Road**
**Wayne Pennsylvania 19087(US)**
Inventor: **Hung, Paul Porwen**
**506 Ramblewood Drive**
**Bryn Mawr Pennsylvania 19010(US)**

(74) Representative: **Brown, Keith John Symons et al**
**c/o John Wyeth & Brother Limited Patent and Trademark Department Huntercombe Lane South**
**Taplow Maidenhead Berkshire SL6 0PH.(GB)**

PROCEEDINGS OF THE NATIONAL ACADEMY OF SCIENCES OF THE USA, vol. 82, no. 5, March 1985, pages 1359-1363, Washington, US; S.L. MANSOUR et al.: "An adenovirus vector system used to express polyoma virus tumor antigens"

PROCEEDINGS OF THE NATIONAL ACADEMY OF SCIENCES OF THE USA, vol. 81, February 1984, pages 1193-1197; R.B. GAYNOR et al.: "Adenovirus early region 1A protein activates transcription of a nonviral gene introduced into mammalian cells by infection or transfection"

CHEMICAL ABSTRACTS, vol. 103, no. 3, 22nd July 1985, page 140, abstract no. 17625a, Columbus, Ohio, US; I. SAITO et al.: "Construction of nondefective adenovirus type 5 bearing a 2.8-kilobase hapatitis B virus DNA near the right end of its genome", & J. VIROL. 1985, 54(3), 711-19

PROCEEDINGS OF THE NATIONAL ACADEMY OF SCIENCES OF THE USA, vol. 79, August 1982, pages 4927-4931; D. PANICALI et al.: "Construction of poxviruses as cloning vectors: Insertion of the thymidine kinase gene from herpes simplex virus into the DNA of infectious vaccinia virus"

NUCLEAR ACIDS RESEARCH, vol. 12, no. 4, 1984, pages 1925-1941, IRL Press Ltd., Oxford, GB; K.L. BERKNER et al.: "Expression of dihydrofolate reductase, and of the adjacent Elb region in an Ad5-dihydrofolate reductase recombinant virus"

DNA Tumor Viruses, J. Tooze (Ed.), CSH, 1980, pp. 388-393; 398-399; 514-517; 930-933; 452-453

## Description

A major goal of biomedical research is to provide protection against viral disease through immunization. One approach has been to use killed vaccines. However, large quantities of material are required for killed vaccine in order to retain sufficient antigenic mass. In addition, killed vaccines are often contaminated with undesirable products during their preparation. Heterologous live vaccines, using appropriately engineered adenovirus, which is itself a vaccine, Chanock, R.M. et al., JAMA, 195, 151 (1966), seem excellent immunogens. Our invention concerns live oral vaccines using adenovirus as vector.

Presently marketed adenovaccine comprises live, infectious adenoviruses in an enteric-coated dosage form. Upon administration to the patient to be vaccinated, the virus is carried past the upper respiratory system (where disease-producing infection is thought to occur), and is released in the intestine. In the intestine, the virus reproduces in the gut wall, where although it is not capable of causing adenoviral disease, it nevertheless induces the formation of adenovirus antibodies, thus conferring immunity to adenoviral disease. In our invention, live, infectious adenovirus which has been engineered to contain genes coding for antigens produced by other disease causing organisms are administered in an enteric-coated dosage form. Upon release in the intestine the virus will reproduce in the gut wall, inducing the formation of antibodies or inducing cell mediated immunity to both adenovirus and the other disease-causing organism. By "live virus" is meant, in contradistinction to "killed" virus, a virus which is, either by itself or in conjunction with additional genetic material, capable of producing identical progeny. By "infectious" is meant having the capability to deliver the viral genome into cells.

Approximately 200,000 persons in the United States are infected each year with Hepatitis B virus. In addition, there is a strong correlation between hepatitis B infection and liver cancer. The presently marketed vaccines against hepatitis B are injectable products manufactured from the blood plasma of healthy carriers by a lengthy process involving several discrete steps.

There are two major hepatitis B viral antigens; the surface antigen (HB$_s$Ag) and the core antigen (HB$_c$Ag). The antigenic structure of HB$_s$Ag is somewhat complex. There is a common group-specific determinant, a. In addition, there are two sets of mutually exclusive type-specific determinants d or y and w or r. The HB$_c$Ag is of a single antigenic type. It is known that production of antibody against HB$_s$Ag or HB$_c$Ag confers immunity against hepatitis B infection.

Several groups have employed recombinant DNA techniques to synthesize the HB$_s$Ag by microorganisms. HB$_s$Ag has been synthesized in Escherichia coli in the form of a fusion protein (Edman, J.C. et al., Nature, 291, 503 (1981)). It has also been synthesized in yeast using the ADH promoter (Valenzuela et al., Nature, 298, 347 (1982)) or acid phosphatase promoter (Miyanohara et al., Proc. Natl. Acad. Sci. USA, 80, 1 (1983)). Finally, vaccinia virus has been used as a vector to produce a live virus vaccine to hepatitis virus (Smith et al., Nature, 302, 490 (1983)).

Rotaviruses are a major cause of acute gastroenteritis in infants. These viruses possess a genome of eleven double-stranded RNA segments enclosed in a capsid. The capsid contains an inner and outer shell. One of the outer shell proteins, VP7, is a glycoprotein that reacts with serotype-specific neutralizing antibodies (Kalica, A.R. et al., Virology, 112, 385 (1981)). This protein is coded for by gene 9 of the human type 1 (Wa) rotavirus. Gene 9 of type 1 human rotavirus has recently been cloned in E. coli and its sequence determined (Richardson, M. et al., J.Virol., 51, 860 (1984)). Thus far, there has been no report of the expression of this protein in any other system.

Adenoviruses contain a linear duplex DNA molecule (m.w. 20 x 10$^6$ - 25 x 10$^6$) that codes for 20-30 polypeptides. Many of these are incorporated into the viral particle which is morphologically complex and has a sophisticated assembly process. Previously SV40 T antigen has been expressed using an adenovirus recombinant (Solnick, D.Cell, 24, 135 (1981), Thummel, C. et al., Cell, 23, 825 (1981), Gluzman, Y. et al., in Eukaryotic Viral Vectors, p. 187, Cold Spring Harbor (1982)). Also mouse dihydrofolate reductose has been expressed using an adenovirus recombinant (Berkner, K. and Sharp,P.A.,Nucleic Acids Research, 12,1925 (1984)). "DNA Tumor Viruses", Cold Spring Harbor Monograph Series, Part 2, 1980, edited by J. Tooze at, for example, pages 388-390, 392-393, 398 and 515-516 discloses the classification of human adenoviruses and restriction maps showing the location of sequences for the coding of various polypeptides. More up-to-date restriction maps are given in, for example "The Adenoviruses" (Harold S Ginsberg Ed.) Plenum Press, New York 1984.

WO-A-8302393 (Animal Vaccine Research Corp.) discloses viruses (eg. adenoviruses) containing protein fragments for example, antigenic functions. The viral surface protein is modified to contain within its structure a portion having the foreign antigemic function.

The invention concerns a method for producing antibodies or cell mediated immunity to an infectious organism in a warm-blooded animal which comprises orally administering to said warm-blooded animal, in an enteric coated dosage form, live recombinant adenoviruses containing the gene coding for the antigen corresponding to said antibodies or inducing said cell mediated immunity. In particular, the invention concerns a method for producing antibodies to hepatitis-B virus or rotavirus in a warm-blooded animal which comprises orally administering to said warm-blooded animal live recombinant adenoviruses containing the gene coding for, respectively, a hepatitis-B antigen or a rotavirus antigen.

In one aspect the invention provides a vaccine for producing antibodies or cell mediated immunity to an infectious organism in warm-blooded animals comprising live recombinant human adenoviruses of type 3, 4, 5 or 7 which contain the gene coding for the antigen which corresponds to said antibodies or induces said cell mediated immunity, said gene coding being present in the viral genome in the E3 region, in the E4 region or at the right hand end of the genome between the TATA box of the E4 promoter and the inverted terminal repeat, said vaccine being formulated in an enteric coated dosage form.

Particularly provided is a vaccine for producing antibodies to hepatitis-B or rotavirus in warm-blooded animals, comprising live recombinant adenoviruses which contain the gene coding for, respectively, a hepatitis-B antigen or a rotavirus antigen, said vaccine being formulated in an enteric-coated dosage form.

The invention also provides a method of preparing a vaccine for producing antibodies or cell mediated immunity to an infectious organism in warm-blooded animals which comprises producing live recombinant human adenoviruses of type 3, 4, 5 or 7, by inserting into adenoviruses the gene coding for the antigen which corresponds to said antibodies or induces said cell mediated immunity, said gene coding being present in the viral genome in the E3 region, in the E4 region or at the right hand end of the genome between the TATA box of the E4 promoter and the inverted terminal repeat, and placing the live recombinant adenoviruses in an enteric coated dosage form.

The invention particularly relates to the use in a vaccine of a recombinant adenovirus which contains a gene coding for an antigen produced by a disease-causing organism said recombinant adenovirus being of type 4, 5, or 7; the gene codes being for hepatitis-B surface antigen or rotavirus outer shell protein; and the gene being located at deleted early region 3.

In the following "Detailed Description of the Invention" mention is made of the gene coding being in the early region 1. This is for reference purposes only. According to the invention the gene coding is in the E3 region, in the E4 region or at the right hand end of the genome between the TATA box of the E4 promoter and the inverted terminal repeat

Detailed Description of the Invention

1. Adenovirus Vectors

Three adenovirus vectors (Gluzman, Y. et al., in Eukaryotic Viral Vectors p. 187, Cold Spring Harbor Laboratories, 1982) can easily be constructed. To maximize the length of foreign DNA that can be inserted, two expendable regions of the viral genome may be deleted, early region 1 or early region 3 (E1 and E3), or both, of the adenovirus type 5 viral genome. Δ E1 is created by an in vivo recombinational event between a plasmid and a modified adenoviral DNA. (All plasmids described in this specification are propagated in E. coli). The plasmid is formed by insertion of adenoviral DNA sequences between 0 and 17 map units into pBR322 and subsequently, using restriction endonuclease digestion and ligation, deleting sequence between 1.4 and 9.1 map units and placing an XbaI restriction site at this junction. This plasmid is denoted in the art as pAC. The modified adenoviral DNA which contains a single XbaI restriction site at 4.0 map coordinates is formed as follows. XbaI cleaves wild type Ad5 DNA at four sites: 4, 29, 79 and 85 map units. Modified DNA lacking sites at 29, 79 and 85 is isolated by cutting Ad5 DNA with XbaI, transfecting the DNA and isolating the modified adenovirus which lacks XbaI sites at positions 29 and/or 79. This procedure is repeated again and modified adenovirus is isolated containing only the XbaI site at position 4. Such modified adenoviruses can also be readily constructed using techniques of oligonucleotide-directed mutagenesis (Smith, M., and Gillam, S. (1981) in Genetic Engineering, Setlow, J.K. and Hollsender, A., Eds Vol. 3 pp. 1-32, Plenum, New York). In this technique the XbaI restriction sites are destroyed using chemically synthesized oligonucleotides designed to produce silent changes in the amino acid coding regions defined by the respective XbaI restriction endonuclease sites. Vector Δ E3 is constructed by deletion of E3 region sequences. Two modified adenoviruses (type 5) are formed by the procedures outlined above. One contains no XbaI sites, the other contains only the XbaI sites at map coordinates 29, 79, and 85. The left half of the modified DNA containing no XbaI sites is joined with the right half of the modified DNA containing XbaI sites at 79 and 89, forming a

modified adenovirus containing XbaI sites at only 79 and 85 map coordinates. Cleavage of this DNA with XbaI followed by religation forms the Δ E3 viral DNA deleting the E3 region between 79 and 85 map coordinates and placing a single XbaI cloning site at this junction. Δ E1 Δ E3 may be constructed by deletions in both regions in a similar manner.

In a fashion similar to construction of Δ E1, Δ E3, and Δ E1 Δ E3 vectors of adenovirus type 5, vectors of adenovirus types 4 and 7 are formed. For example, in adenovirus type 7, the E3 region is deleted between the XbaI site at 80.5 map coordinates and the EcoRI site at map coordinate 85.

2. 6X Series Plasmids. (For $HB_sAg$ and rotavirus VP7)

Plasmids that allow the placement of the adenovirus 2 late promoter upstream from DNA coding for hepatitis-B surface antigen or rotavirus VP7 followed by SV40 splicing signals may be constructed. Each of these is flanked by XbaI sites for insertion into the adenovirus Δ E1, Δ E3, or Δ E1E3 vectors.

a. p6XH

Plasmid 6XH contains an XbaI linker at -400bp of the Ad2 major late promoter and an EcoRI site at +33bp, 8bp before the end of the first adenovirus late leader. This is followed by an Eco RI linker at 26bp preceding the ATG of $HB_sAg$ followed by $HB_sAg$ sequence of 678bp to another EcoRI linker at 809bp. This is followed by SV40 sequence extending from 2753-2516 bp on the SV40 map. These sequences are all inserted into the large pBR 322 Bam HI to EcoRI fragment via XbaI linkers.

b. p6XR

Plasmid p6XR is made by joining the rotavirus VP7 gene with ECoRI linkers at nucleotide 6 and 1036 to the Ad2 major late promoter containing an XbaI site at -400 bp and an Eco RI linker at +331 and attaching SV40 sequences from 2753-2516 behind the VP7 gene with an Eco RI linker at 2753 (SV40 map coordinate) and an XbaI site at 2516 bp. This cassette is inserted into the large Eco RI to BamH fragment of pBR322.

c. Transfer of plasmid sequences to the viral DNA vector and production of recombinant adenovirus.

The transfer of the cassette of promoter foreign gene-terminator to the adenovirus vector is done either as follows or by in vivo recombination (see detailed example below). The purified adrenovirus vector DNA is cleaved with a restriction endonucleose followed by treatment with calf intestine alkaline phosphatase to prevent self ligation. Plasmid derived sequences are obtained by cleavage of p6XH or p6XR with XbaI. These are then ligated to the adenoviral vector DNA. Either 293 cells, (Grahm, et al., Gen. Virol., 86 10 (1978)), Hela cells, or Wi38 cells are then transfected with the ligation mixture and overlayed with agar. Plaques are picked 7-10 days later and viral stocks prepared.

3. Expression Assays.

Three types of assays have been used to assess expression of hepatitis-B surface antigen and rotavirus VP7. These are:

a. Indirect immunofluorescence.

Either mouse monoclonal antibodies or rabbit antisera are used to detect expression of recombinant Δ E1 and Δ E3 virus stocks containing $HB_sAg$ or VP7 DNA sequences. Counterstaining is with goat anti-mouse or anti-rabbit FITC.

b. Immunoprecipitation.

Immunoprecipitation of $HB_sAg$ or rotavirus VP7 in cells infected with the recombinant adenoviruses is done using either mouse monoclonal antibodies or rabbit polyclonal antisera against $HB_sAg$ or rotavirus VP7 and protein A Sepharose CL4B.

c. RIA

Expression of $HB_sAg$ is also tested by a commercially available radioimmunoassay (Ausria, Abbott Labs.).

4. Immunogenic Nature of the Recombinant Adenovirus.

Live, lyophilized recombinant adenovirus contained in an enteric coated capsule is assessed for immunogenicity by giving them ($10^4$ - $10^5$ 50% infectious dose/tablet) to hamsters or chimpanzees and testing for antibody levels and protection from challenge.

The presently marketed adenovirus vaccine contains living lyophillized adenovirus of either type 4 or type 7 mixed with inert ingredients prepared in enteric coated tablets. Administration of tablets (approximately $10^4$ $TCID_{50}$ of virus) results in selective gastrointestinal infection without illness. The vaccine is safe; the induced infection is specifically restricted to the intestinal tract, and the vaccine virus is not transmitted from vaccinees to susceptible close contacts. Specific neutralizing antibody is noted in over 95% of vaccinated individuals 21 days after immunization. The new vaccines of the present invention which are specifically described are comprised of recombinant adenoviruses expressing hepatitis-B surface antigen and rotavirus VP7 and are formulated and work in the same fashion as the present adenovirus vaccine except that antibody to hepatitis-B surface antigen or rotavirus VP7 is produced as well as antibody to adenovirus. In any of the embodiments of the invention, the administration of approximately $10^4$ $TCID_{50}$ of recombinant virus, or even considerably less, will, of course, produce the desired immunogenic response. The determination of the optimum dosage will vary depending on the particular recombinant adenovirus employed; determination of this optimum is within the skill of the art.

5. Detailed example of a recombinant that expresses authentic $HB_sAg$.

As a detailed example of the construction of one adenovirus recombinant, the $HB_sAg$ gene of the adw subtype from 26 bp upstream of the $HB_sAg$ translation initiation codon and 131 bp downsteam from the translation termination codon was flanked by upstream sequences from the Ad2 major late promoter (+33 to 400 bp; Solnick, D., Cell, 24, 135-143 (1981) and by downstream sequences from SV40 (2753 to 2516 bp; Tooze, J. (Ed.) Molecular Biology of Tumor Viruses, Cold Spring Harbor Laboratory pp. 801-829 (1980)). This plasmid is termed p6XH (see above). These sequences were inserted into the unique XbaI site plasmid pAC that contains an insert of Ad5 DNA extending from the Eco RI linker at the left end of the adenovirus genome to the Hind III site at Ad5 map coordinate 17.0 (Gluzman, Y., Reichl, H., and Solnick, D., 1982, in (Y. Gluzman, Ed.) Eukaryotic Viral Vectors, Cold Spring Harbor Laboratories, p. 187-192). The new plasmids (pACH-2 and pACH-9) with the cassette containing the Ad2 major late promoter-$HB_sAg$ gene-SV40 processing signals in either orientation, were cleaved with Hind III. The Hind III cleavage product of each was combined with the large XbaI fragment of the modified adenovirus $\Delta$ E1 extending from map coordinates 9.1 to 100 (Gluzman, Y., Reichl, H., and Solnick, D., 1982 in (Y. Gluzman, ed.) Eukaryotic Viral Vectors, Cold Spring Harbor Laboratories, pp 187-192). This DNA mixture was transfected (Graham, F.L. and ven der Eb, A.J. Virology 52, 456-467 (1973) onto 293 cells (Graham, F.L., Smiley, J., Russell, W.C., and Nairn, R., J. Gen. Virol., 36, 59-72 (1977))and cells were overlaid with agar for plaque detection. Approximately 10-14 days later, 54 plaques were picked and virus stocks generated from each. These viruses were screened for the presence of $HB_sAg$ DNA by hybridization to a $^{32}$P-labelled $HB_sAg$ DNA probe. Positive plaques (49 out of 54) were next infected onto monolayers of 293 cells and the expression of authentic $HB_sAg$ was detected in cell lysates by both radioimmunoassay (AUSRIA, Abbot Laboratories, Inc. or NML-$HB_sAg$ RIA, Nuclear-Medical Laboratories) and by immunoprecipitation of $^{35}$S-radiolabelled $HB_sAg$ using a monoclonal antibody to $HB_sAg$ (anti-a subtype, Boehringer Mannheim Biochemicals).

6. In the specific example above, we use the Ad2 major late promoter extending only 33 nucleotides downstream from the transcriptional initiation site so that the first splice site is not included. This promoter contains only the first part of the tripartite leader of the adenoviral major late promoter. However, the major later promotor from other adenoviruses particularly types 3,4,5, or 7 can be used and the full tripartite leader of this promoter can be used. In the following example we describe the construction of two bacterial plasmids, pHM1 and pHM2 which contain cassettes composed of the Ad2 major late promoter and the leftmost 168 bp of the 200 bp Ad2 tripartite leader followed by the $HB_sAg$ gene and processing and polyadenylation signals from SV40 virus. These plasmids also contain adenovirus sequences flanking the cassette so that homologous recombination can be used to insert the cassette into the adenoviral genome. On the left the cassette is flanked by the leftmost 353 bp of the Ad5 genome and on the right by map coordinates 8-15.5 of adenovirus 5. Plasmid pHM1 contains 19 bp of SV40 virus sequence (SV40 nucleotides 5173-5174) preceding the $HB_sAg$ gene. Plasmid pHM2 is identical to pHM1 except that it does not contain this sequence.

The cassettes from both pHM1 and pHM2 were placed at the E1 region of the adenovirus 5 genome by the technique of homologous recombination as described above. Each plasmid was linearized and combined with the large XbaI fragment of the adenovirus mutant $\Delta$ E1 extending from map coordinates 9.1 to 100 (Gluzman, Y., Reichl, H. and Solnick, D., 1982 in (Y. Gluzman, ed.) Eukaryotic Viral Vectors, Cold Spring Harbor Laboratories, pp. 187-192). Plaques were picked and stock viruses generated from each.

When these stock viruses (HM1 and HM2) were infected on a human embryonic kidney (293) cell line (Graham, F.L., Smiley, J., Russell, W.C. and Nairn, R. (1977) J. Gen. Virol. 36 , 59-72) we found, after 40 h infection, approximately 1 $\mu$g $HB_sAg$ (based upon radioimmunoassay and comparison of cpm to NML-$HB_sAg$ kit positive control) per 5 x $10^6$ infected cells were observed in culture supernatants of HM2 infected cells. HM1 virus yielded approximately 60% of this amount. We found that the $HB_sAg$ polypeptides produced by these viruses were glycosylated (P2) and non-glycosylated (P1) forms (Marion, P.L., Salazar, F.H., Alexander, J.J. and Robinson, W. (1979) J. Virol. 32, 796-802; Peterson D.L. (1981) J. Biol. Chem. 256, 6975-6983). At 40 h after infection most of the HB$_s$Ag (78%) was secreted from cells into the culture medium as particle (density = 1.20 g/ml) the same or nearly the same as the 22 nm particle (Gerin, J.L., Purcell, R.H., Hoggan, M.D., Holland, P.V. and Chanock, R.M. (1969) J. Virol. 4, 763-768; Gerin, J.L., Holland, P.V., and Purcell, R.H. (1971) J.Virol. 7, 569-576) observed in human serum. HM2 yielded approximately 40% more HB$_s$Ag than HM1. However, when HM2 was compared to the previously described hybrid adenovirus, Δ E1H, a 70-fold increase in HB$_s$Ag polypeptide was noted by HM2 virus.

Instead of the adenoviral major late Promoter, any other suitable eukaryotic promoter can be used, such as human metallothionein promoter or the human dihydrofolate reductase promoter. In addition, in our examples, we have used adenovirus type 5 DNA as vector for foreign gene expression; however, other adenovirus types can be used as vector, and particularly useful are types 3, 4 and 7 that are presently in use as vaccines.

Also, we describe the use of processing and polyadenylation signals from SV40 DNA; however, any suitable processing and polyadenylation signals may be used. These may come from adenoviral DNA, particularly types 3, 4 and 7.

In practising the method of this invention, where the foreign gene is inserted in deleted early region 3 of the adenovirus, the recombinant virus remains infective, and the vaccination requires nothing more than delivery of the recombinant virus to the gut. On the other hand, early region 1 is essential to adenovirus infectivity. Therefore, if the foreign gene is inserted in deleted early region 1, helper virus must be co-administered. This helper virus is conveniently unmodified, infectious adenovirus. Also, the helper virus can itself be a defective virus with a deletion which can be complemented by the recombinant virus. In this fashion virus growth and foreign antigen production would be elicited only in cells infected with both viruses. This defective helper can be of the same subtype as, or of different subtype to, the recombinant virus. If of differing subtype (e.g. if recombinant virus was Ad4 subtype and the defective virus of Ad7 subtype), formation of wild type virus through recombination should be minimized. Propagation of virus for vaccine production can be accomplished either through co-cultivation of both viruses in human diploid fibroblasts or cultivation of viruses separately in cell lines known to complement each of the defects.

In addition to the E1 and E3 regions, there are several other regions of the viral genome where the cassette containing promoter, tripartite leader, foreign gene, and processing and polyadenylation signals may be inserted. This include a region between E1a and E1b, regions at the right end of the genome, and in the E4 region.

Some examples are given below:

Ad5 contains an E1a promoter at map coordinate 1.4 and an E1b promoter at map coordinate 4.7. The polyadenylation site for E1a is at map coordinate 4.6, nucleotide 1631, at nucleotide 1671 is the TATA box unique Hpal site (GTTAAC). This site can be utilized for placement of the adenovirus type 2 major late promoter and hepatitis-B surface antigen and use of the E1a polyadenylation site. Polyadenylation of E1a can be provided by placement of a polyadenylation signal from SV40 viral DNA behind E1a or from L4 region of the virus. The additional DNA in the genome in the above construct may be compensated by removal of DNA determined non-essential in the E3 region.

Other insertion points are the extreme right end of the genome. At the right end the position will be between the 116 bp inverted terminal repeat and the TATA box of the E4 promoter. In Ad2 there is an Mbo II site at 99.3 map units. This is 191 bp from the extreme right end of the viral DNA. In Ad5 there is a Tha1 (FnU4DII) site 240 bp from the right end of the genome. This region is between the ITR and upstream of the E4 TATA box. Again, if necessary, insertion will be made into an E3 deletion adenovirus to accommodate the extra DNA.

In each case these same regions can be used as insertion points for the cassette of the adenovirus major late promoter, adenovirus tripartite leader, foreign gene and procesing and polyadenylation signals in adenovirus type 4 and type 7 strains that are used for the presently marketed adenovirus vaccines. Adenovirus of types 4 or 7 are preferred for use in the invention since these are the types presently employed in commercial adenovirus vaccines. Although specific reference has been made to vaccines producing antibodies to hepatitis-B and rotavirus, our invention provides oral vaccines against any infectious agent containing an antigen to which a warm-blooded animal will produce antibodies or cell mediated immunity, and which antigen is coded for by a gene composed of up to about 3000 base pairs. Thus, for example, included within the scope of the invention are immunization against such diseases as influenza, parainfluenza, respiratory synctial virus disease, hepatitis A, acquired immunodeficiency syndrome (AIDS), cholera, E. coli, pertussis, diphtheria, tetanus, shigellosis, gonorrhea, mycoplasma pneumonia, and so on.

## Claims

**Claims for the following Contracting States: BE, CH, DE, FR, IT, LI, LU, NL, SE**

1. A vaccine for producing antibodies or cell mediated immunity to an infectious organism in warm-blooded animals comprising live recombinant human adenoviruses of type 3, 4, 5 or 7 which contain the gene coding for the antigen which corresponds to said antibodies or induces said cell mediated immunity, said gene coding being present in the viral genome in the E3 region, in the E4 region, or at the right hand end of the genome between the TATA box of the E4 promoter and the inverted terminal repeat, said vaccine being formulated in an enteric coated dosage form.

2. A vaccine according to Claim 1 wherein said infectious organism is the hepatitis-B virus.

3. A vaccine according to Claim 2 wherein the gene codes for hepatitis-B surface antigen.

4. A vaccine according to Claim 3 wherein said live recombinant adenovirus is adenovirus type 4 or 7 with the gene located in deleted early region 3.

5. A vaccine according to Claim 1 wherein the infectious organism is the rotavirus.

6. A vaccine according to Claim 5 wherein the gene codes for rotavirus outer shell protein.

7. A vaccine according to Claim 6 wherein said live recombinant adenovirus is adenovirus type 4 or 7 with the gene located in deleted early region 3.

8. A vaccine according to Claim 1 wherein the recombinant adenovirus is defective, and the vaccine also comprises a second adenovirus containing the gene region which is missing in the defective recombinant adenovirus.

9. A vaccine according to Claim 1 wherein the recombinant adenovirus is of type 4, 5 or 7.

10. A method of preparing a vaccine as claimed in claim 1 for producing antibodies or cell mediated immunity to an infectious organism in warm-blooded animals which comprises producing live recombinant human adenoviruses of type 3, 4, 5 or 7, by inserting into adenoviruses the gene coding for the antigen which corresponds to said antibodies or induces said cell mediated immunity, the gene coding being inserted is the viral genome in the E3 region, in the E4 region or at the right hand end of the genome between the TATA box of the E4 promoter and the inverted terminal repeat, and placing the live recombinant adenoviruses in an enteric coated dosage form.

**Claims for the following Contracting State: AT**

1. A method of preparing a vaccine for producing antibodies or cell mediated immunity to an infectious organism in warm-blooded animals which comprises formulating live recombinant human adenoviruses of type 3, 4, 5 or 7 which contain the gene coding for the antigen which corresponds to said antibodies or induces said cell mediated immunity, said gene coding being present in the viral genome in the E3 region, in the E4 region, or at the right hand end of the genome between the TATA box of the E4 promoter and the inverted terminal repeat, in an enteric coated dosage form.

2. A method according to Claim 1 wherein said infectious organism is the hepatitis-B virus.

3. A method according to Claim 2 wherein the gene codes for hepatitis-B surface antigen.

4. A method according to Claim 3 wherein said live recombinant adenovirus is adenovirus type 4 or 7 with the gene located in deleted early region 3.

5. A method according to Claim 1 wherein the infectious organism is the rotavirus.

6. A method according to Claim 5 wherein the gene codes for rotavirus outer shell protein.

7. A method according to Claim 6 wherein said live recombinant adenovirus is adenovirus type 4 or 7 with the gene located in deleted early region 3.

8. A method according to Claim 1 wherein the recombinant adenovirus is defective, and the vaccine also comprises a second adenovirus containing the gene region which is missing in the defective recombinant adenovirus.

9. A method according to Claim 1 wherein the recombinant adenovirus is of type 4, 5 or 7.

**Patentansprüche**
**Patentansprüche für folgende Vertragsstaaten:**
**BE, CH, DE, FR, IT, LI, LU, NL, SE**

1. Vakzine zum Produzieren von Antikörpern oder zellvermittelter Immunität auf einen infektiösen Organismus in Warmblütern, die lebende rekombinante menschliche Adenoviren vom Typ 3, 4, 5 oder 7 umfaßt, welche das Gen codierend für das Antigen enthält, das diesen Antikörpern entspricht, oder diese zellvermittelte Immunität induziert, wobei das codierende Gen im viralen Genom in der E3-Region, in der E4-Region oder am rechten Ende des Genoms zwischen der TATA-Box des E4-Promotors und der invertierten terminalen Sequenzwiederholung vorhanden ist, welche Vakzine in einer enterisch-überzogenen Dosierungsform formuliert ist.

2. Vakzine nach Anspruch 1, in der der infektiöse Organismus das Heptatitis B-Virus ist.

3. Vakzine nach Anspruch 2, in der das Gen für Hepatitis B-Oberflächenantigen codiert.

4. Vakzine nach Anspruch 3, in der das lebende rekombinante Adenovirus vom Adenovirus-Typ 4 oder 7 ist, wobei sich das Gen in der deletierten frühen Region 3 befindet.

5. Vakzine nach Anspruch 1, in der der infektiöse Organismus das Rotavirus ist.

6. Vakzine nach Anspruch 5, in der das Gen für Rotavirus-Außenhüllprotein codiert.

7. Vakzine nach Anspruch 6, in der das lebende rekombinante Adenovirus vom Adenovirus-Typ 4 oder 7 ist, wobei sich das Gen in der deletierten frühen Region 3 befindet.

8. Vakzine nach Anspruch 1, in der das rekombinante Adenovirus unvollständig ist und die Vakzine auch ein zweites Adenovirus umfaßt, das die Genregion enthält, die im unvollständigen rekombinanten Adenovirus fehlt.

9. Vakzine nach Anspruch 1, in der das rekombinante Adenovirus vom Typ 4, 5 oder 7 ist.

10. Verfahren zum Herstellen einer Vakzine nach Anspruch 1 zum Produzieren von Antikörpern oder zellvermittelter Immunität auf einen infektiösen Organismus in Warmblütern, das das Produzieren lebender rekombinanter menschlicher Adenoviren vom Typ 3, 4, 5 oder 7 durch Insertieren des Gens codierend für das Antigen, das diesen Antikörpern entspricht oder diese zellvermittelte Immunität induziert, in Adenoviren umfaßt, wobei das insertierte codierende Gen das virale Genom in der E3-Region, in der E4-Region oder am rechten Ende des Genoms zwischen der TATA-Box des E4-Promotors und der invertierten terminalen Sequenzwiederholung ist, welche Vakzine in einer enterisch-überzogenen Dosierungsform formuliert ist.

**Patentansprüche für folgenden Vertragsstaat: AT**

1. Verfahren zum Herstellen einer Vakzine zum Produzieren von Antikörpern oder zellvermittelter Immunität auf einen infektiösen Organismus in Warmblütern, das das Formulieren lebender rekombinanter menschlicher Adenoviren vom Typ 3, 4, 5 oder 7 enthaltend das Gen, das für das Antigen codiert, welches diesen Antikörpern entspricht oder diese zellvermittelte Immunität induziert, wobei das codierende Gen im viralen Genom in der E3-Region, in der E4-Region oder am rechten Ende des Genoms zwischen der TATA-Box des E4-Promotors und der invertierten terminalen Sequenzwiederholung vorhanden ist, in einer enterischüberzogenen Dosierungsform.

2. Verfahren nach Anspruch 1, in dem der infektiöse Organismus das Heptatitis B-Virus ist.

3. Verfahren nach Anspruch 2, in dem das Gen für Hepatitis B-Oberflächenantigen codiert.

4. Verfahren nach Anspruch 3, in dem das lebende rekombinante Adenovirus vom Adenovirus-Typ 4 oder 7 ist, wobei sich das Gen in der deletierten frühen Region 3 befindet.

5. Verfahren nach Anspruch 1, in dem der infektiöse Organismus das Rotavirus ist.

6. Verfahren nach Anspruch 5, in dem das Gen für Rotavirus-Außenhüllprotein codiert.

7. Verfahren nach Anspruch 6, in dem das lebende rekombinante Adenovirus vom Adenovirus-Typ 4 oder 7 ist, wobei sich das Gen in der deletierten frühen Region 3 befindet.

8. Verfahren nach Anspruch 1, in dem das rekombinante Adenovirus unvollständig ist und die Vakzine auch ein zweites Adenovirus umfaßt, das die Genregion enthält, die im unvollständigen rekombinanten Adenovirus fehlt.

9. Verfahren nach Anspruch 1, in dem das rekombinante Adenovirus vom Typ 4, 5 oder 7 ist.

**Revendications**
**Revendications pour les Etats contractants suivants: BE, CH, DE, FR, IT, LI, LU, NL, SE**

1. Vaccin produisant des anticorps ou une immunité à médiation cellulaire contre un organisme infectieux chez les animaux à sang chaud, comprenant des adénovirus humains recombinants vivants de type 3, 4, 5 ou 7 qui contiennent le gène codant l'antigène qui correspond auxdits anticorps ou induit ladite immunité à médiation cellulaire, ledit gène codant étant présent dans le génome viral dans la région E3, dans la région E4, ou à l'extrémité du côté droit du génome entre la boîte TATA du promoteur E4 et la répétition terminale inversée, ledit vaccin étant formulé dans une forme dosée à enrobage entérique.

2. Vaccin selon la revendication 1, dans lequel ledit organisme infectieux est le virus de l'hépatite B.

3. Vaccin selon la revendication 2, dans lequel le gène code l'antigène de surface de l'hépatite B.

4. Vaccin selon la revendication 3, dans lequel ledit adénovirus recombinant vivant est l'adénovirus de type 4 ou 7 avec le gène situé dans la région 3 précoce délétée.

5. Vaccin selon la revendication 1, dans lequel ledit organisme infectieux est le rotavirus.

6. Vaccin selon la revendication 5, dans lequel le gène code une protéine de l'enveloppe extérieure du rotavirus.

7. Vaccin selon la revendication 6, dans lequel l'adénovirus recombinant vivant est un adénovirus de type 4 ou 7 avec le gène situé dans la région 3 précoce délétée.

8. Vaccin selon la revendication 1, dans lequel l'adénovirus recombinant est défectif et le vaccin comprend aussi un deuxième adénovirus contenant la région du gène qui est manquante dans l'adénovirus recombinant défectif.

9. Vaccin selon la revendication 1, dans lequel l'adénovirus recombinant est de type 4, 5 ou 7.

10. Procédé de préparation d'un vaccin selon la revendication 1 pour produire des anticorps ou une immunité à médiation cellulaire contre un organisme infectieux chez les animaux à sang chaud, qui comprend la production d'adénovirus humains recombinants vivants de type 3, 4, 5 ou 7 par insertion dans les adénovirus du gène codant l'antigène qui correspond auxdits anticorps ou induit ladite immunité à médiation cellulaire, le gène codant étant inséré dans le génome viral dans la région E3, dans la région E4 ou à l'extrémité du côté droit du génome entre la boîte TATA du promoteur E4 et la répétition terminale inversée, et l'introduction des adénovirus recombinants vivants dans une forme dosée à enrobage entérique.

**Revendications pour l'Etat contractant suivant: AT**

1. Procédé de préparation d'un vaccin pour produire des anticorps ou une immunité à médiation cellulaire contre un organisme infectieux chez les animaux à sang chaud, qui comprend la formulation d'adénovirus humains recombinants vivants de type 3, 4, 5 ou 7 qui contiennent le gène codant l'antigène qui correspond auxdits anticorps ou induit ladite immunité à médiation cellulaire, ledit gène codant étant présent dans le génome viral dans la région E3, dans la région E4, ou à l'extrémité du côté droit du génome entre la boîte TATA du promoteur E4 et la répétition terminale inversée, dans une forme dosée à enrobage entérique.

2. Procédé selon la revendication 1, dans lequel ledit organisme infectieux est le virus de l'hépatite B.

3. Procédé selon la revendication 2, dans lequel le gène code l'antigène de surface de l'hépatite B.

4. Procédé selon la revendication 3, dans lequel l'adénovirus recombinant vivant est un adénovirus de type 4 ou 7, avec le gène situé dans la région 3 précoce délétée.

5. Procédé selon la revendication 1, dans lequel l'organisme infectieux est le rotavirus.

6. Procédé selon la revendication 5, dans lequel le gène code une protéine de l'enveloppe extérieure du rotavirus.

7. Procédé selon la revendication 6, dans lequel l'adénovirus recombinant vivant est un adénovirus de type 4 ou 7, avec le gène situé dans la région 3 précoce délétée.

8. Procédé selon la revendication 1, dans lequel l'adénovirus recombinant est défectif, et le vaccin comprend aussi un deuxième adénovirus contenant la région du gène qui est manquante dans l'adénovirus recombinant défectif.

9. Procédé selon la revendication 1, dans lequel l'adénovirus recombinant est du type 4, 5 ou 7.